# EUROPEAN PATENT SPECIFICATION

(11) **EP 1 184 374 B1**
(45) Date of publication and mention of the grant of the patent: **28.01.2009**
(21) Application number: 01203872.5
(22) Date of filing: 29.04.1997
(51) Int. Cl.: C07D 211/60, C07D 401/06, C07D 401/12, A61K 31/435

(54) **Carboxamide derivatives of pyrrolidine, piperidine and hexahydroazepine for the treatment of thrombosis disorders**
Carboxamidderivate von Pyrrolidin, Piperidin und Hexahydropiperizin für Behandlung von Thrombosen
Derivés carboxamide de pyrrolidine, pipéridine et hexahydroazépine utilisés dans le traitement de troubles thrombotiques

(30) Priority: 01.05.1996 US 16675 P
(43) Date of publication of application: 06.03.2002
(62) Divisional of application: 97922518.2
(73) Proprietor: Ortho-McNeil Pharmaceutical, Inc., Raritan, NJ 08869-0606 (US)
(72) Inventor: Costanzo, Michael, J., Ivyland, PA 18974 (US); Hoekstra, William, J., Villonova, PA 19085 (US); Maryanoff, Bruce, E., Forest Grove, PA 18922 (US)
(74) Representative: Mercer, Christopher Paul

(56) References cited:
- WO-A-95/08536
- WO-A-95/11228
- WO-A-95/25091
- WO-A-96/29309
- DE-A- 4 326 344
- HOEKSTRA, WILLIAM J. ET AL: "Design and Evaluation of Nonpeptide Fibrinogen.gamma. Chain-Based GPIIB/IIIA Antagonists" J. MED. CHEM. (1995), 38(10), 1582-92 CODEN: JMCMAR;ISSN: 0022-2623, 1995, XP000572765
- HOEKSTRA, WILLIAM J. ET AL: "Solid-phase parallel synthesis applied to lead optimization: discovery of potent analogs of the GPIIb/IIIa antagonist RWJ-50042" BIOORG. MED. CHEM. LETT. (1996), 6(20), 2371-2376 CODEN: BMCLE8;ISSN: 0960-894X, 16 October 1996 (1996-10-16), XP002039034

## Description

### BACKGROUND OF THE INVENTION

Platelet aggregation constitutes the initial hemostatic response to curtail bleeding induced by vascular injury. However, pathological extension of this normal hemostatic process can lead to thrombus formation. The final, common pathway in platelet aggregation is the binding of fibrinogen to activated, exposed platelet glycoprotein IIb/IIIa (GPllb/llla). Agents which interrupt binding of fibrinogen to GPIIb/IIIa, therefore, inhibit platelet aggregation. These agents are, therefore, useful in treating platelet-mediated thrombotic disorders such as arterial and venous thrombosis, acute myocardial infarction, unstable angina, reocclusion following thrombolytic therapy and angioplasty, inflammation, and a variety of vaso-occlusive disorders. The fibrinogen receptor (GPIIb/IIIa) is activated by stimuli such as ADP, collagen, and thrombin exposing binding domains to two different peptide regions of fibrinogen: α-chain Arg-Gly-Asp (RGD) and γ-chain His-His-Leu-Gly-Gly-Ala-Lys-Gln-AJa-Gly-Asp-Val (HHLGGAKQAGDV, γ400-411). Since these peptide fragments themselves have been shown to inhibit fibrinogen binding to GPIIb/IIIa, a mimetic of these fragments would also serve as an antagonist In fact, prior to this invention, potent RGD-based antagonists have been revealed which inhibit both fibrinogen binding to GPllb/Illa and platelet aggregation e.g., Ro-438857 (L Alig, J. Med. Chem. 1992, 35, 4393) has an IC₅₀ of 0.094 µM against in vitro thrombin-induced platelet aggregation. Some of these agents have also shown *in vivo* efficacy as antithrombotic agents and, in some cases, have been used in conjunction with fibrinolytic therapy e.g., t-PA or streptokinase, as well (J. A. Zablocki, Current Pharmaceutical Design 1995, 1, 533). As demonstrated by the results of the pharmacological studies described hereinafter, the compounds of the present invention show the ability to block fibrinogen binding to isolated GPIIb/IIa (IC₅₀'s 0.0002-1.39 µM), inhibit platelet aggregation *in vitro* in the presence of a variety of platelet stimuli (0.019-65.0 µM vs. thrombin), and furthermore, inhibit *ex vivo* platelet aggregation in animal models. Additionally, these agents exhibit efficacy in animal thrombosis models as their progenitors had shown ("Nipecotic Acid Derivatives As Antithrombotic Compounds," application Serial No. 08/213772, filed March 16, 1994). The compounds of the present invention show efficacy as antithrombotic agents by virtue of their ability to prevent platelet aggregation. Additionally, because the compounds of this invention inhibit integrin-mediated cell-cell or cell-matrix adhesion, they may also be useful against inflammation, bone resorption, tumor cell metastasis, etc. (D. Cox, Drug News&Perspectives 1995, 8, 197).

DE 43 26 344 A1 discloses platelet aggregation inhibitors. WO 95/08536 discloses β-alanine derivatives as glycoprotein II/b IIIa antagonists, inhibitors of blood platelet aggregation and inhibitors of the binding of fibrinogen to blood platelets. WO 95/25091 discloses nipecotic acid-derived compounds useful in treating platelet-mediated thrombotic disorders. W-J. Hoekstra et. al., J. Med. Chem. 1995, 38, 1582-1592 discloses design and evaluation of nonpeptide fibrinogen X-chain based glycoprotein IIb/IIIa antagonists.

### DISCLOSURE OF THE INVENTION

The present invention is directed to compounds as defined in claim 1.

These platelet aggregation inhibitors are useful in treating platelet-mediated thrombotic disorders such as arterial and venous thrombosis, acute myocardial infarction, reocclusion following thrombolytic therapy and angioplasty, inflammation, unstable angina, and a variety of vaso-occlusive disorders. These compounds are also useful as antithrombotics used in conjunction with fibrinolytic therapy (e.g., t-PA or streptokinase). Pharmaceutical compositions containing such compounds are also part of the present invention.

### DETAILED DESCRIPTION OF THE INVENTION

More particularly, the present invention is directed to compounds as defined in claim 1.

This disclosure also relates to pharmaceutically acceptable salts of the compounds of the present invention. The pharmaceutically acceptable salt generally takes a form in which the nitrogen on the 1-piperidine (pyrrolidine, piperazine) substituent is protonated with an inorganic or organic acid. Representative organic or Inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benezenesulfonic, oxalic, pamoic, 2-naphthalonesulfonic, *p*-toluenesultonic, cyclohexanesulfamic, salicyilc, saccharinic or trifluoroacetic.

The compounds of the present invention are those compounds shown in Table 1, where "Subst" indicates the position of attachment of the group C(O)N(R¹)YCO₂H to the central azacycle. As these numerals do not have any configuration specified this indicates that the compounds are racemic mixtures.

**TABLE I**

| | | | | | | | | |
|---|---|---|---|---|---|---|---|---|
| 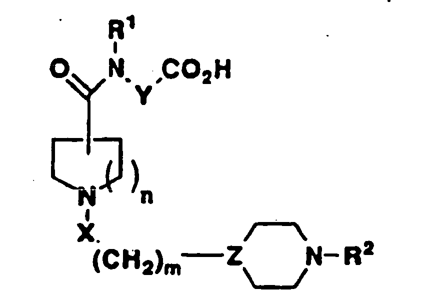 | | | | | | | | |

| # | Subst | m | n | X | R¹ | R² | **Y** | Z |
|---|---|---|---|---|---|---|---|---|
| **2** | 3 | 1 | 2 | NHCO | H | H | CH₂CHMe | CH |
| **3** | 3 | 1 | 2 | OC(O) | H | H | (*R*)-CH(CO₂Me)CH₂ | CH |
| **12** | 3 | 2 | 2 | SO₂ | H | H | CH₂CH₂ | CH |

The compounds of the invention may be prepared using the methodology exemplified in Scheme AA. In this scheme nipecotic acid allyl ester (either the racemic mixture or either separate enantiomer) may be treated with resin-bound 4-piperidinepropionic acid in the presence of DIC/HOBT and a tertiary amine. The allyl ester is then removed via palladium-mediated catalysis and the iterative coupling process continued to give final product upon saponification with potassium trimethylsilanolate (e.g., synthesis compound 1). By analogy, urea and urethane-based replacements for the tertiary amide (compounds **2** and **3** of the present invention) were prepared by reaction of solid-supported amine (alcohol) with *p*-nitrophenylchloroformate and then ethyl nipecotate (S. M. Hutchins, Tetrahedron Lett. 1994, 35, 4055).

Three-substituted 3-aminopropionic acid ester intermediates were prepared utilizing a modified Knoevenagel procedure (Scheme AG; E. Profft, J. Prakt. Chem. 1965, 30. 18) followed by Fischer esterification of the carboxylic acid product (when not commeraally-available). These intermediates were prepared in enantiomerically-enriched form by penicillin amidase resolution of racemic phenylacetamides such as intermediate AG3 (V. A. Soloshonok, Tetrahedron: Asymmetry 1995, 6, 1601). Here, the undesired R-enantiomer is hydrolyzed by amidase while the desired S-enantiomer retains the phonylacetyl group. Resolutions may also be performed on the (-)-ephedrine salts of racemic three-substituted 3-N-Boc-aminopropionic acids as published (J. A. Zablocki, J. Med. Chem. 1995, 38, 2378). Ethyl nipecotate and ethyl isonipecotate are commercially-available intermediates.

Synthesis of 5- and 7-membered ring analogues of nipecotamides (**4** and **17**, respectively) were prepared by solid-phase synthesis using methyl pyrrolidine-3-carboxylate and methyl hexahydroazepine-3-carboxylate intermediates for the analogous conversion of AA2 to AA3 (Scheme AA). Methyl pyrrolidine-3-carboxylate and methyl hexahydroazepine-3-carboxylate were prepared as published (H. Rapoport, J. Org. Chem. 1974, 39, 893). For example, N-benzyl hexahydroazepin-2-one was reacted with lithium diisopropylamide/diethyicarbonate and this product then reduced with lithium aluminum hydride to afford N-benzyl-3-hydroxymethylhexahydroazepine. The benzyl group was removed by hydrogenolysis (H₂. Pd-C, MeOH), the nitrogen protected (di-*t*-butyldicarbonate/sodium hydroxide), and the alcohol oxidized with chromium trioxide to give N-Boc-hexahydroazepine-3-carboxylic acid. The Boc group was removed concomitant with carboxylate esterification using HCl/MeOH to afford methyl hexahydroazepine-3-carboxylate.

Sulfonamide **12** was prepared as shown in Scheme AE. Intermediate AE1 was isolated in two steps from 4-pyridineethanesulfonic acid by hydrogenation/protection as described (J. I. DeGaw, J. Heterocyclic Chem. 1966, 3, 90), and then chlorinated using standard thionyl chloride conditions (P. J. Hearst, Org. Syn. 1950, 30, 58) to give AE2. Intermediate AE2 was then carried forward to final product using standard solution-phase synthesis (W. J. Hoekstra, J. Med. Chem 1995, 38, 1582).

Enantiomerically-enriched R-(-)-nipecotic acid ethyl ester was isolated by chiral revolution of racemic material as its corresponding D-tartaric acid salt (A. M. Akkerman, Rec Trav. Chirp. Pays-Bas 1951, 70, 899)

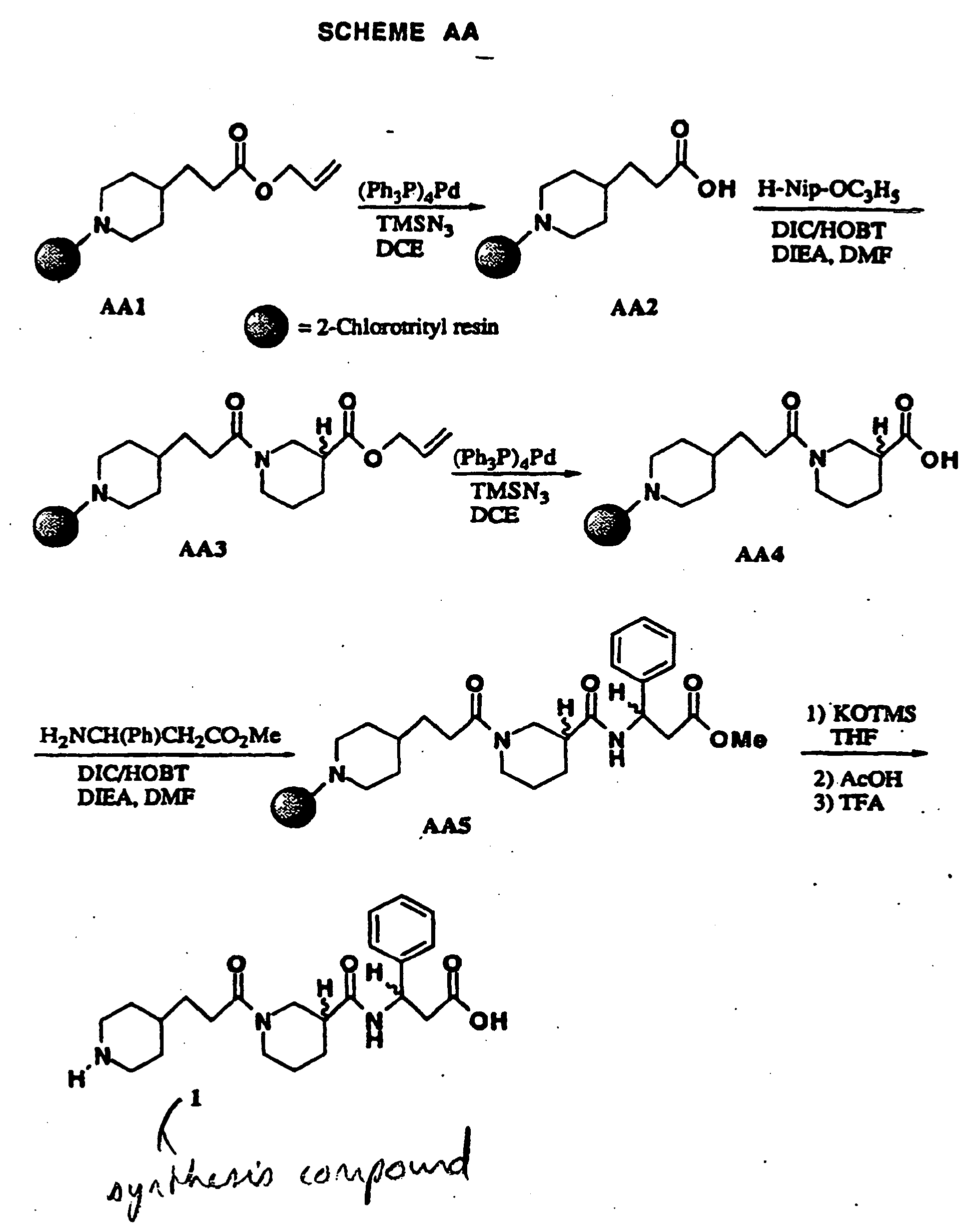

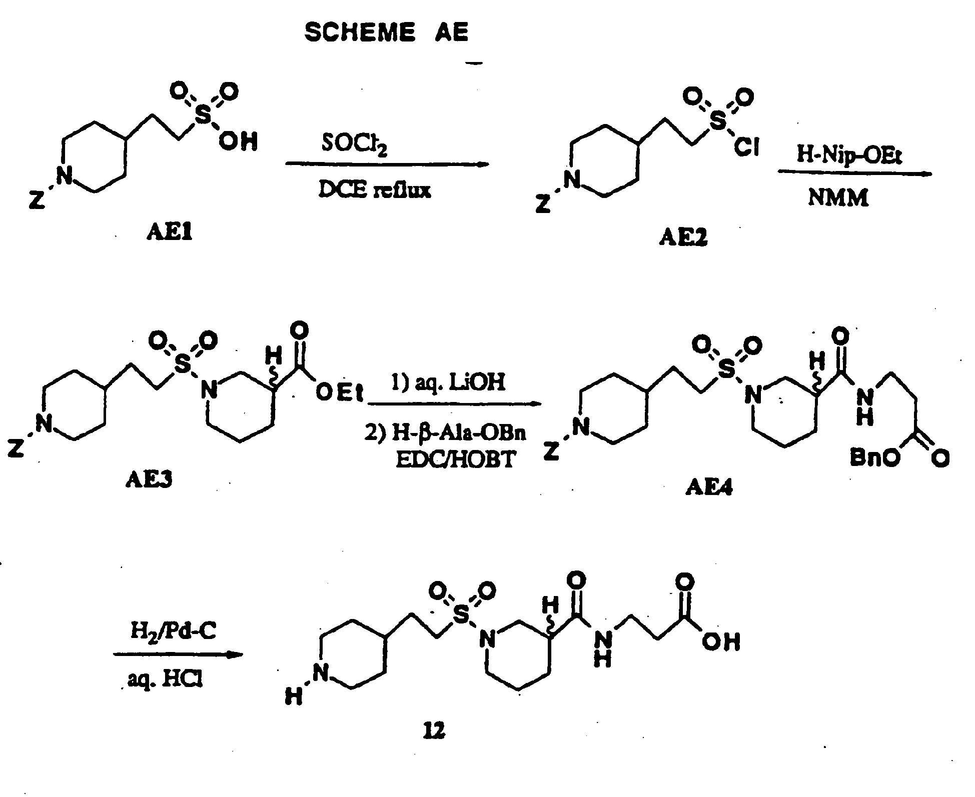

To prepare the pharmaceutical compositions of this invention, one or more compounds or salt thereof of the invention as the active ingredient, is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.03 mg to 100 mg/kg (preferred 0.1-30 mg/kg) and may be given at a dosage of from about 0.1-300 mg/kg/day (preferred 1-50 mg/kg/day). The dosages, however, may be varied depending upon the requirement of the patients, the severity of the condition being treated and the compound being employed. The use of either daily administration or post-periodic dosing may be employed.

### BIOLOGY

The compounds of the present invention interrupt binding of fibrinogen to platelet glycoprotein IIb/IIIa (GPIIb/IIIa) and thereby inhibit platelet aggregation. Such compounds are, therefore, useful in treating platelet-mediated thrombotic disorders such as arterial and venous thrombosis, acute myocardial infarction, reocclusion following thrombolytic therapy and angioplasty, and a variety of vaso-oclusive disorders. Because the final, common pathway in normal platelet aggregation is the binding of fibrinogen to activated, exposed GPII/bIIIa, inhibition of this binding represents a plausible antithrombotic approach. The receptor is activated by stimuli such as ADP, collagen, and thrombin, exposing binding domains to two different peptide regions of fibrinogen: α-chain Arg-Gly-Asp (RGD) and γ-chain 400-411. As demonstrated by the results of the pharmacological studies described hereinafter, the compounds of the present invention show the ability to block fibrinogen binding to isolated GPllb/lla (IC₅₀'s 0.0002-1.39 µM), inhibit platelet aggregation *in vitro* in the presence of a various of platelet stimuli (0.019-65.0 µM vs. thrombin), and furthermore, inhibit *ex vivo* platelet aggregation in animal models.

### IN VITRO SOLID PHASE PURIFIED GLYCOPROTEIN IIB/IIIA BINDING ASSAY.

A 96 well Immulon-2 microtiter plate (Dynatech-Immulon) is coated with 50 µl/well of RGD-affinity purified GPIIb/IIIa (effective range 0.5-10 µg/mL) in 10 mM HEPES. 150 mM NaCl. 1 mM at pH 7.4. The plate is covered and incubated overnight at 4°C. The GPII/bIIIa solution is discarded and 150 µl of 5% BSA is added and incubated at RT for 1-3 h. The plate is washed extensively with modified Tyrodes buffer. Biotinylated fibrinogen (25 µl/well) at 2 x final concentration is added to the wells that contain the test compounds (25 µl/well). The plate is covered and incubated at RT for 2-4 h. Twenty minutes prior to incubation completion, one drop of Reagent A (Vecta Stain ABC Horse Radish Peroxidase kit, Vector Laboratories. Inc.) and one drop Reagent B are added with mixing to 5 mL modified Tyrodes buffer mix and let stand. The ligand solution is discarded and the plate washed (5 x 200 µ/well) with modified Tyrodes buffer. Vecta Stain HRP-Biotin-Avidin reagent (50 µl/well, as prepared above) is added and incubated at RT for 15 min. The Vecta Stain solution is discarded and the wells washed (5 x 200 µ/well) with modified Tyrodes buffer. Developing buffer (10 mL of 50 mM citrate/phosphate buffer @ pH 5.3. 6 mg o-phenylenediamine, 6 µl 30% H₂O₂; 50 µl/well) is added and incubated at RT for 3-5 min, and then 2*N* H₂SO₄ (50 µl/well) is added. The absorbance is read at 490 nM. The results are shown in Tables III and IV.

### IN VITRO INHIBITION OF THROMBIN-INDUCED GEL-FILTERED PLATELET AGGREGATION ASSAY.

The percentage of platelet aggregation is calculated as an increase in light transmission of compound-treated platelet concentrate vs. control-treated platelet concentrate. Human blood is obtained from drug free, normal donors into tubes containing 0.13*M* sodium citrate. Platelet rich plasma (PRP) is collected by centrifugation of whole blood at 200 x g for 10 min at 25°C. The PRP (5 mL) is gel filtered through Sepharose 2B (bed volume 50 mL), and the platelet count is adjusted to 2x10⁷ platelets per sample. The following constituents are added to a siliconized cuvette: concentrated platelet filtrate and Tyrode's buffer (0.14*M* NaCl. 0.0027*M* KCl, 0.012M NaHCO₃. 0.76 *mM* Na₂HPO₄, 0.0055M glucose, 2 mg/mL BSA and 5.0*mM* HEPES @ pH 7.4) in an amount equal to 350 µl. 50 µl of 20 *mM* calcium and 50 µl of the test compound. Aggregation is monitored in a BIODATA aggregometer for the 3 min following the addition of agonist (thrombin 50 µl of 1 unit/mL). The results are shown in Table III.

**TABLE III**

| **In Vitro Results** | | | | |
|---|---|---|---|---|
| | Fibrinogen Binding | | Platelet Aggregation* | |
| Compound # | % Inh, (50 µM) | IC₅₀ (µM) | % lnh. (50µM) | IC₅₀ (µM) |
| **2** | 93.0% | 0.027 | 95.7% | 54.0 |
| **3** | 81.0% | NT | 26.2% | >100 |
| **12** | 97.0% | 0.025 | 88.0% | 15.5 |

| | | | | |
|---|---|---|---|---|
| *Thrombin-induced aggregation of gel-filtered platelets. | | | | |

### EXAMPLES

Protected amino acids were purchased from Aldrich Chemical or Bachem Bioscience Inc. 2-Chlorotrityl resin and Wang resin were obtained from Novabiochem Corp. EnantiomerieWly-enriched cycloalkylidene-3-carboxylic acid ethyl esters were isolated by chiral resolution of racemic material as published (A. M. Akkerman. Rec. Trav. Chim. Pays-Bas 1951, 70, 899). All other chemicals were purchased from Aldrich Chemical Company. Inc. Final product acid addition salts can be converted to free bases by basic ion exchange chromatography. High field ¹H NMR spectra were recorded on a Bruker AC-360 spectrometer at 360 MHz, and coupling constants are given in Herz. Melting points were determined on a Mel-Temp II melting point apparatus and are uncorrected. Microanalyses were performed at Robertson Microlit Laboratories, Inc., Madison, New Jersey. In those cases where the product is obtained as a saft, the free base is obtained by methods known to those skilled in the art, *e.g*. by basic ion exchange purification. In the Examples and throughout this application, the following abbreviations have the meanings recited hereinafter.
Bn or Bzl = Benzyl
Boc = t-Butoxycarbonyl
BOC-ON = 2-(t-Butoxycarbonyloxyimino)-2-phenylacetonitrile
BOP-Cl = Bis(2-oxo-3-oxazolidinyl)phosphinic chloride
CP = compound
DCE = 1,2-Dichloroethane
DCM = Dichloromethane
DIBAL-H = Diisobutylaluminum hydride
DIC - Diisopropylcarbodiimide
DIEA - Dlisopropytethylamine
DMAP = 4-Dimethylaminopyridine
DMF = N, N-Dimethylformamide
EDC = Ethyl dimethylaminopropylcarbodiimide
EDTA = Ethylenediaminetetraacetic acid
Et₂O = Diethyl ether
HBTU = 2-(1H-Beruotriazote-1-yl)-1,t,3,3-tetramethyluronium hexafluorophosphate
HOBT = Hydroxybenzotriazole
*i*-Pr = Isopropyl
KOTMS = Potassium trimethylsilanolate
NMM = N-Methylmorpholine
Nip = Nipecotyl (unless noted otherwise, racemic at 3-position)
NT = not tested -
PPT = precipitate
PTSA = *p-*Toluenesulfonic acid
RT = room temperature
TFA = Trifluoroacetic acid -
TMSN₃ = Azidotrimethylsilane
Z = Benzyloxycarbonyl

### Allyl 3-(4-piperidine)propionate • HCl (AA1 precursor)

To a mixture of 3-(4-pyridine)acrylic acid (10.0 g, 0.066 mol) and aqueous HCl (2.0 *N*, 50 mL) under a blanket of nitrogen was added platinum (IV) oxide (0.54 g). This mixture was hydrogenated at 50 psi and RT for 21 h, filtered through Celite, and evaporated to give 3-(4-piperidine)propionic acid • HCI as a white powder (12.9 g, 99%). This powder was treated with allyl alcohol (50 mL) and warmed at 50°C for 2 h. This solution was cooled to RT, evaporated to *ca*. 10 mL volume, and diluted with Et₂O (250 mL). The resultant precipitate was collected and washed with Et₂O to afford a white powder (14.5 g, 94%): ¹H NMR (DMSO-d₆) δ 8.7-9.1 (m, 2 H), 5.9 (m, 1 H), 5.25 (dd, J=7,15, 2 H), 4.53 (d, J=4, 2 H), 3.21 (d, J=8, 2 H), 2.74 (t. J=7. 2 H), 2.35 (t, J=4, 2 H), 1.72 (d, J=8, 2 H). 1.5 (m. 3 H), 1.3 (m. 2 H); MS m/e 198 (MH⁺). The aqueous layer was adjusted to pH 2 (conc. HCl), extracted with Et₂O, and evaporated to a white foam. The foam was purified by silica gel chromatography (10% MeOH/DCM) to give AG3. A solution of compound AG3 (0.22 mol) in water (600 mL) at RT was adjusted to pH 7.5 using KOH (3.0 *N)* and treated with penicillin amidase (91520 units, Sigma). This mixture was stirred for 47 h, acidified to pH 1 with HCl (conc), and the resultant ppt filtered through Celite. The filtrate was extracted with Et₂O (3x300 mL), concentrated *in vacuo,* and treated with MeOH/conc. NH₄OH (9:1), This product-containing solution was purified by silica gel chromatography (eluent DCM/MeOH/NH₄OH, 78:18:4) to give (S)-3-phenylacetamido-3-(3-pyridyl) propionic acid ammonium salt (19.5 g, 58%). This product was treated with HCl (6.0 (N, 292 mL), heated at reflux for 5 h, cooled to RT, and extracted with Et₂O (3x200 mL). The aqueous layer was adjusted to pH 12, concentrated *in vacuo,* and the resultant solid triturated with MeOH (2x300 mL). This solution was evaporated to give *ca*. 14 g sodium salt. This material was treated with MeOH (500 mL), 2,2-dimethoxypropane (44 mL), and HCl (4 N in dioxane, 84 mL), and stirred for 90 h at RT. This mixture was filtered and the filtrate concentrated *in vacuo.* The resultant off-white solid was triturated with Et₂O (2 x 150 mL) and dried to give compound AG5 (16.7 g, 96% ee) as a white, amorphous solid.

### EXAMPLE 1 (Comparative)

### N-3-(4-Piperidinepropionyl)-nipecotyl-(3-amino-3-phenyl) propionic acid • TFA (1)

A 25 mL sintered glass vessel under nitrogen was charged with 2-chlorotrityl chloride resin (0.24 g, 0.36 mmol, Novabiochem) and DMF (5 mL). The resin was agitated with nitrogen for 5 min to swell and the DMF removed. The resin was treated with DMF (5 mL), DIEA (0.31 mL, 5 eq), and allyl 3-(4-piperidine)propionate • HCl (0.20 g, 2.4 eq), sequentially, and agitated for 8 h. The resultant dark green solution was removed, and the resin washed with DMF (3x5 mL), aqueous DMF (25%, 3x5 mL), THF (3x5 mL), DCM (3x5 mL), and Et₂O (5 mL). The resin was swelled with DCE (5 mL) and treated with a mixture of tetrabutylammonium fluoride hydrate (0.28 g, 3 eq), azidotrimethylsilane (0.38 mL, 10 eq), tetrakis(triphenylphosphine)palladium (0.084 g, 20 mol %), and DCE (5 mL). The resin was agitated for 15 h and the orange solution removed. The resin was washed with DCM (3x5 mL), DMF (3x5 mL), THF (3x5 mL), and Et₂O (5 mL). The resin was swelled with DMF (5 mL) and treated with DIEA (0.18 mL, 3 eq), allyl nipecotate • HCl (0.17 g, 3 eq), DIC (0.17 mL, 3 eq), and HOBT (1 mg). The resin was agitated for 15 h and then the reaction solution removed. The resin was washed with DMF (3x5 mL), aqueous DMF (25%, 3x5 mL), THF (3x5 mL), DCM (3x5 mL), and Et₂O (5 mL). The resin was swelled with DCE (5 mL) and treated with a mixture of tetrabutylammonium fluoride hydrate (0.28 g, 3 eq), azidotrimethylsilane (0.38 mL, 10 eq), tetrakis(triphenylphosphine) palladium (0.084 g, 20 mol %), and DCE (5 mL). The resin was agitated for 15 h and the orange solution removed. The resin was washed with DCM (3x5 mL), DMF (3x5 mL), THF (3x5 mL), and Et₂O (5 mL). The resin was swelled with DMF (5 mL) and treated with DIEA (0.18 mL, 3 eq), methyl D,L-3-amino-3-phenylpropionate • HCl (0.23 g, 3 eq), DIC (0.17 mL, 3 eq), and HOBT (1 mg). The resin was agitated for 17 h and then the reaction solution removed. The resin was washed with DMF (3x5 mL), aqueous DMF (25%. 3x5 mL), THF (3x5 mL), DCM (3x5 mL), and Et₂O (5 mL). The resin was swelled with THF (5 mL) and treated with a solution of KOTMS (0.23 g, 10 eq) and THF (2 mL). The resin was agitated for 18 h and then the reaction solution removed. The resin was washed with DMF (3x5 mL), acetic acid/THF (1:1, twice), aqueous DMF (25%, 3x5 mL), THF (3x5 mL), DCM (3x5 mL), and Et₂O (5 mL). The resin was treated with TFA/DCM (1:1, 10 mL), agitated for 15 min, and the resultant red solution collected. This solution was evaporated and the resultant oil triturated with Et₂O (3x5 mL) and dried to afford compound 1 as a dear glass (0.11 g): ¹H NMR (DMSO-d₆) δ 8.6 (m, 1 H), 8.42 (d, J=7, 1H), 8.2 (m, 1H), 7.3 (m, 3 H), 7.2 (m, 2 H), 5.18 (d, J=6, 1H), 4.3 (m, H),3.7(m, 1 H). 3.2 (m, 3 H), 2.8 (m, 2 H). 2.6 (m. 2 H), 2.3 (m, 5 H), 1.1-1.9 (m, 11 H); MS m/e 416 (MH⁺).

Using the same general solid phase synthesis technique as described in Example 1, the compounds of indicated examples were made according to Scheme AA as recited in the particular example.

### EXAMPLE 2

### N-(4-Piperidinemethylaminocarbonyl)-nipecotyl-(3-amino-2-methyl) propionic acid • TFA (2)

Compound **2** was prepared as shown in Scheme AA. Resin-bound 4-piperidinemethylamine (0.36 mmol) was swelled with DCE (5 mL), treated with *p*-nitrophonylchloroformate (0.36 mmol) and DIEA (0.36 mmol), agitated for 1 h. and the solvent removed. The resin was washed (see Example 1), swelled with DCE (5 mL), treated with allyl nipecotate • HCl (0.36 mmol) and DIEA (0.72 mmoL), and agitated for 16 h. The solvent was removed, the resin washed (see Example 1), and the allyl ester cleaved to the corresponding acid (see Example 1). The resin was swelled with DMF (5 mL), the acid coupled with methyl 3-amino-2-methylproplonate (0.36 mmol), and the synthesis completed as shown in Example 1. Compound 2 was isolated as a dear glass (0.11 g): ¹H NMR (CD₃OD) δ 3.9 (m, 2 H), 3.2 (m, 4 . H), 3.10 (d, J=7,2 H), 2.9 (m, 3 H), 2.6 (m, 2 H), 2.3 (m, 1 H), 1.9 (m, 4 H), 1.7-1.9 (m, 5 H), 1.3-1.5 (m, 5 H), 1.11 (d, J=7, 3 H); MS m/e 355 (MH⁺).

### EXAMPLE 3

### N-(4-Piperidinemethyloxycarbonyl)-nipecotyl-D-aspartic acid α-methyl ester • TFA (3)

Compound **3** was prepared as shown in Scheme AA. Resin-bound 4-piperidinemethanol (0.36 mmol) was swelled with DCE (5 mL), treated with p-nitrophenylchloroformate (0.36 mmol) and DIEA (0.36 mmol), agitated for 1 h, and the solvent removed. The resin was washed (see Example 1), swelled with DCE (5 mL), treated with allyl nipecotate • HCl (0.36 mmol) and DIEA (0.72 mmoL), and agitated for 16 h. The solves was removed, the resin washed (see Example 1), and the allyl ester cleaved to the corresponding acid (see Example 1). The resin was swelled with DMF (5 mL), the acid coupled with H-D-Asp(OBn)-OMe (0.36 mmol), and the synthesis completed as shown in Example 1. Compound 3 was isolated as a yellow glass (0.019 g): ¹H NMR (CD₃OD) δ 4.8 (m, 2 H), 3.9 (m, 3 H), 3.70 (d. J=9, 4 H), 3.39 (s, 3 H), 3.3 (m, 2 H), 2.9 (m, 4 H), 2.8 (m, 2 H), 1.9 (m, 4 H), 1.7 (m, 2 H), 1.4 (m, 4 H); MS m/e 400 (MH⁺).

### EXAMPLE 4

### N-3-(4-Piperidineethanesulfonyl)-nipecotyl-3-aminopropionic acid • HCl (12)

Compound 12 was prepared as shown in Scheme AE. Intermediate AE1 was synthesized by the following procedure. 2-(4-Pyridine)ethanesulfonic acid (3.0 g, 0.016 mol) was dissolved in aq. HCl (2.0 N, 12 mL) and this solution treated with platinum dioxide (0.13 g) and hydrogenated at 50 psi and RT for 18 h. This mixture was filtered through Celite and evaporated to afford 2-(4-piperidine)ethanesulfonic acid · HCl (3.5 g, white powder). This powder was dissolved in aq. THF (1:1, 70 mL) at RT and treated with NMM (3.7 mL, 2.2 eq.) and benzyl chloroformate (2.2 mL, 1 eq.). This mixture was stirred for 15 h**,** acidified with aq. citric add, and extracted with CHCl₃ (2x100 mL). The organic layer was dried with Na₂SO₄ and evaporated to afford 2-(4-N-Z-piperidine)ethanesulfonic acid (2.75 g, gold oil). This oil was converted to final product **12** in five synthetic steps (Scheme AE, W. J. Hoekstra. J. Med. Chem. 1995, 38, 1582) and isolated as a clear glass (0.060 g): ¹H NMR (DMSO-d₆) δ 8.9 (m, 1H), 8.6 (m, 1 H), 3.5 (m, 2 H), 3.1-3.3 (m, 4 H), 3.0 (m, 2 H), 2.6-2.8 (m, 4 H), 2.3 (m, 3 H), 1.65-1.9 (m, 5 H), 1.6 (m, 3 H), 1.2-1.4 (m, 5 H): MS m/e 376 (MH⁺).

## Claims

1. A compound represented by the following formula: 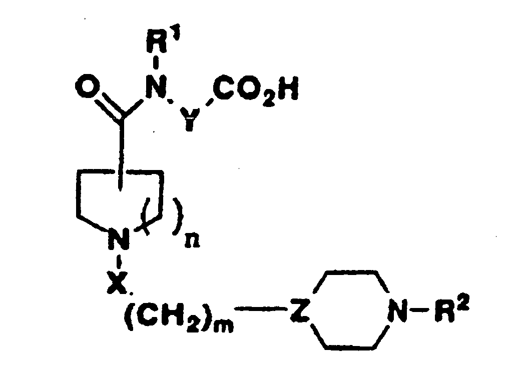 wherein
the C(O)N(R¹)YCO₂H group is attached to the central azacycle at the 3 position;
the value of n is 2;
R¹ is H;
R² is H;
Z is CH; and
m, X and Y are selected from one of the following three combinations:
m is 1, X is NHCO and Y is CH₂CHMe;
m is 1, X is OC(O) and Y is (*R*)-CH(CO₂Me)CH₂; and
m is 2, X is SO₂ and Y is CH₂CH₂.

2. A compound of claim 1 for use in treating platelet-mediated thrombotic disorders.

3. A composition for treating platelet-mediated thrombotic disorders comprising the compound of claim 1 in an effective amount for treating such disorders in combination with a pharmaceutically acceptable carrier.

4. The composition of claim 3, wherein the amount is 0.1-300 mg/kg/day.

## Patentansprüche

1. Verbindung, dargestellt durch die folgende Formel: 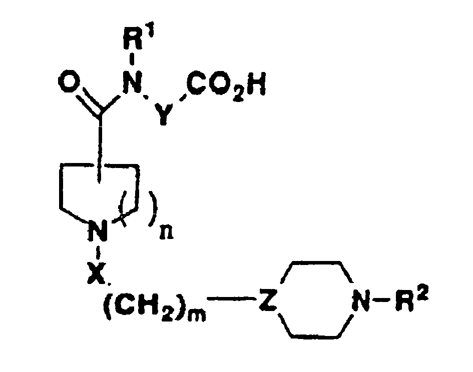 worin
die C(O)N(R¹)YCO₂H-Gruppe an den zentralen Azacyclus an der 3-Position gebunden ist;
der Wert von n 2 ist;
R¹ H ist;
R² H ist;
Z CH ist; und
m, X und Y ausgewählt sind aus einer der folgenden drei Kombinationen:
m ist 1, X ist NHCO und Y ist CH₂CHMe;
m ist 1, X ist OC(O) und Y ist (*R*)-CH(CO₂Me)CH₂; und
m ist 2, X ist SO₂ und Y ist CH₂CH₂.

2. Verbindung nach Anspruch 1 zur Verwendung bei der Behandlung von Thrombozyten-vermittelten thrombotischen Störungen.

3. Zusammensetzung zur Behandlung von Thrombozyten-vermittelten thrombotischen Störungen, die die Verbindung nach Anspruch 1 in einer wirksamen Menge zur Behandlung solcher Störungen in Kombination mit einem pharmazeutisch verträglichen Träger umfasst.

4. Zusammensetzung nach Anspruch 3, wobei die Menge 0,1-300 mg/kg/Tag beträgt.

## Revendications

1. Composé représenté par la formule suivants : 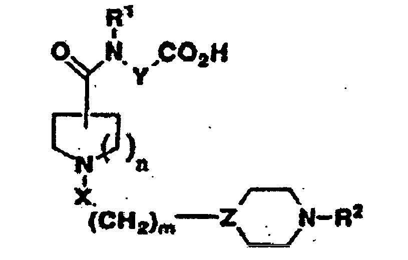 dans laquelle
le groupe C(O)N(R¹)YCO₂H est fixé à l'azacycle central en position 3 ;
la valeur de n est 2 ;
R¹ représente H ;
R² représente H ;
Z représente CH ; et
m, X et Y sont choisis parmi l'une des trois combinaisons suivantes :
m vaut 1, X représente NHCO et Y représente CH₂CHMe ;
m vaut 1, X représente OC(O) et Y représente (*R*)-CH(CO₂Me)CH₂ ; et
m vaut 2, X représente SO₂ et Y représente CH₂CH₂.

2. Composé selon la revendication 1, pour une utilisation dans le traitement de troubles thrombotiques médiés par les plaquettes.

3. Composition destinée à traiter des troubles thrombotiques médiés par les plaquettes comprenant le composé selon la revendication 1 dans une quantité efficace pour traiter de tels troubles en combinaison avec un support pharmaceutiquement acceptable.

4. Composition selon la revendication 3, dans laquelle la quantité est de 0,1 à 300 mg/kg/jour.
